# EUROPEAN PATENT SPECIFICATION

(11) **EP 2 793 966 B1**
(45) Date of publication and mention of the grant of the patent: **28.03.2018**
(21) Application number: 12826677.2
(22) Date of filing: 21.12.2012
(51) Int. Cl.: A61L 27/50, A61L 15/42

(54) **SELECTIVE PLASMA ACTIVATION FOR MEDICAL IMPLANTS AND WOUND HEALING DEVICES**
SELEKTIVE PLASMAAKTIVIERUNG FÜR MEDIZINISCHE IMPLANTATE UND WUNDHEILUNGSVORRICHTUNGEN
ACTIVATION PAR PLASMA SÉLECTIVE POUR IMPLANTS MÉDICAUX ET DISPOSITIFS DE CICATRISATION

(30) Priority: 22.12.2011 US 201161579060 P
(43) Date of publication of application: 29.10.2014
(73) Proprietor: Centre Hospitalier Universitaire Vaudois (CHUV), 1011 Lausanne (CH)
(72) Inventor: PIETRAMAGGIORI, Giorgio, 2010 Lausanne (CH); MAJD, Hicham, 1630 Bulle (CH)
(74) Representative: Cronin, Brian Harold John
(86) International application number: PCT/IB2012/057586
(87) International publication number: WO 2013/093868

(56) References cited:
- WO-A2-2010/026557
- JEAN-PHILIPPE FRIMAT ET AL: "Plasma stencilling methods for cell patterning", ANALYTICAL AND BIOANALYTICAL CHEMISTRY, SPRINGER, BERLIN, DE, vol. 395, no. 3, 17 May 2009 (2009-05-17), pages 601-609, XP019736569, ISSN: 1618-2650, DOI: 10.1007/S00216-009-2824-7

## Description

### FIELD OF THE INVENTION

This invention relates to devices for implantation or for application as a wound dressing and is particularly concerned with treating the surfaces of such devices for increasing cell adhesion and decreasing biofouling and fibrosis (encapsulation). The invention relates to the treated device surfaces, the devices with such treated surfaces, and methods and apparatus for treating the device surfaces.

### BACKGROUND OF THE INVENTION

Implantable medical devices (IMDs) are rapidly revolutionizing medicine. Implantable artificial materials and devices, such as drug delivery systems, pacemakers, artificial joints, and organs play an important role in health care today. In addition to these devices, implantable monitoring devices or "biosensors" have great potential for improving both the quality of care and quality of life.

One of the major problems associated with all types of implants is biocompatibility of the implant with the body, and in particular with the tissue adjacent to the site of the implant. Infection and capsular contraction remain significant clinical problems. IMDs are limited by their reduced biocompatibility: the body recognizes the implant as a foreign material and builds scar tissue around it. Lack of biocompatibility leads to reduced performance of the devices, pain to the patients, high re-operation rates, and augmenting the costs and risks significantly. When sensors, electrodes or drug delivery parts are embedded in scar tissue the function of devices such as pacemakers, implantable defibrillators, insulin pumps, neurostimulators is affected with life threatening consequences.

For example, despite attempts to design implantable biosensors for glucose and other monitoring functions, none developed to date provide pain-free, reliable and continuous monitoring. One reason is that current implantable sensors suffer from a progressive loss of function after relatively short periods of time *in vivo.* This loss in function arises from multiple factors, some of the most important of which include protein adsorption, inflammation, and fibrosis (encapsulation) resulting from tissue trauma at the site of the implant. Fibrosis results in loss of blood vessels at the site of implantation and, therefore, in a reduced access to blood glucose levels. These factors can also interfere with the function of other implants and implantable devices, such as insulin pumps, pacemakers, artificial joints, and artificial organs.

One approach to control the inflammation and fibrosis resulting from tissue trauma at the site of implantation has been to use inert materials such as titanium or single-crystalline alumina, as disclosed in U.S. Pat. No. 4,122,605 to Hirabayashi et al. While suitable for bone or tooth implants, this approach is not useful in more complex prosthetic devices or in biosensors, which requires use of a variety of materials. Another approach has been the use of a porous, outer coating of DACRON or TEFLON, as disclosed in U.S. Pat. No. 4,648,880 to Brauman et al., or with polytetrafluorethylene, as disclosed in U.S. Pat. No. 5,779,734. While suitable for prostheses such as breast implants, such coatings are not practical for prosthetic devices or biosensors having complex geometries. The most commonly-used approach to control tissue responses, particularly inflammation, has been the systemic administration of drugs such as corticosteroids. Such systemic administration can result in side effects such as generalized immunosuppression, bloating, and psychiatric problems, especially over the long term. There accordingly remains a need in the art for apparatus and methods for controlling tissue/implant interactions, particularly for implantable materials, prostheses, and devices such as biosensors.

Plasma treatments can be used to oxidize hydrophobic surfaces, transforming the surface free energy to a hydrophilic state, where cell adhesion and growth are possible (J.-P. Frimat et al., Anal. Bioanal Chem (2009) 395: 601-609.). Oxygen plasma operating under vacuum conditions has been previously used to increase cell adhesion. Stenciling methods in combination with oxygen plasma activation were previously implied to depositing cell adhesion molecules on non-adherent substrates generating patterns of defined design (J.L. Dewez et al., Biomaterials 19 (1998) page 1443, Selective cell adhesion on substrates with defined patterns.), typically with a width in the range of a few tens of µm.

Stencilling methods can also be used with atmospheric pressure air plasma, obviating the need for vacuum systems and a gas supply (J.-P. Frimat et al., 2009, *supra*).

Using a Tesla generator, PDMS, methylated glass and BGPS surfaces were rendered hydrophilic (J.-P. Frimat et al., 2009, *supra*). This reference typically has an array of plasma-treated areas of diameter about 200 µm.
Previous *in vitro* and *in vivo* work on patterned cell-adhesive devices is described in WO 2010/026557. A specific protein deposition pattern was identified in this work: islets that guide cell adhesion and reduce fibrosis around medical implants, independently from the protein used. The cell adhesive pattern does not allow fibroblasts to exert forces on the surface where they attach through the focal adhesions and prevent them from becoming myofibroblasts (the main cell responsible for fibrosis and contraction).

The process described in WO 2010/026557, involved the deposition of cell-adhesive proteins, namely collagen, fibronectin, or vitronectin alone or in combination. This process reduced the fibrotic tissue around the implant but the process is expensive and involves multiple steps taking hours to complete. Moreover, the presence of the deposited proteins of animal origin gave rise to concerns for obtaining acceptance from regulatory authorities.

Certain *in vitro* studies showed that cell adhesion is induced by oxygen or air plasma activation of a substrate. With the aid of a stencil, cell adhesive large areas and cell non-adhesive large areas were created for multiple cell adhesion in specific zones. In particular, it was found that cell adhesion is promoted by the adsorption of proteins (i.e. fibronectin) from a culture media to the specifically activated areas (in preference to the non-activated zones) (J.-P. Frimat et al., 2009, *supra*). Cell patterns on large zones remain stable over 10 days in culture, allowing for a stable cell adhesion *in-vitro* conditions. After selective plasma activation, deposited cells pack within activated tracks with sharply demarcated boundaries, indicating the stability of the highly non-adhesive nature of non-activated regions.

The activated areas in current devices are meant for the adhesion of cell aggregates and are not single cell specific. Such general activated surfaces using relatively large stencil patterns do not control focal adhesions. Thus, current devices cannot prevent encapsulation of the implanted device by fibrotic overgrowth.

A significant advance in the art would follow the discovery of a patterned surface that controls cell-adhesion, growth and differentiation and which is also simple in design and can be produced quickly and inexpensively. If the surface of a device further inhibited, retarded or prevented fibrotic overgrowth of the implanted device, the surface would have far ranging applications. Surprisingly, the present invention provides such a surface.

### SUMMARY OF THE INVENTION

In contrast to prior cell-adhesive surfaces, in various embodiments, the present invention provides a surface with activated small (e.g., microscale) areas in order to control the size of the focal adhesions for each cell, with an effect on cell differentiation.

It is an object of the invention to provide a medical device with a cell-adhesive surface and a method to produce it. The surface of the invention is designed to retard, inhibit or prevent fibrotic growth on the device and to inhibit, or prevent fibrotic encapsulation of the device. The surface of the invention is readily prepared by an efficient process, which involves fewer steps than prior methods of preparing biocompatible surfaces.

In an exemplary embodiment, the invention provides a device configured for implantation or wound dressing in a subject. The device comprises a device surface. The device surface comprises a plurality of first elements. Each of the first elements has a first surface. The device surface also includes a plurality of second elements. Each second element comprises a second surface. Each first surface has a first affinity to water. In an exemplary embodiment, the first surface is hydrophilic, or is more hydrophilic than the second surface. Similarly, each second surface has an affinity for water. In various embodiments, the second surface is hydrophobic, or is more hydrophobic than the first surface. In general, the first affinity and the second affinity are different affinities.

Results of *in vitro* and *in vivo* studies illustrate the importance of the size of the islets in reducing fibrosis and myofibroblasts around implants. The first members of the plurality of a first surface represent areas of adhesion (focal adhesions, i.e., where the cells are attached). In an exemplary embodiment, the islets have length that is less than or equal to about 6µm, a width that is less than or equal to about 2µm and distance between the islets that is less than or equal to about 6 µm. A device surface having first and second surfaces so arranged impaired the formation of myofibroblasts.

The elements on the device surface are sized to control cell adhesion, growth and differentiation. Thus, in an exemplary embodiment, each first element has a length of less than about 6µm and a width of less than about 2µm.

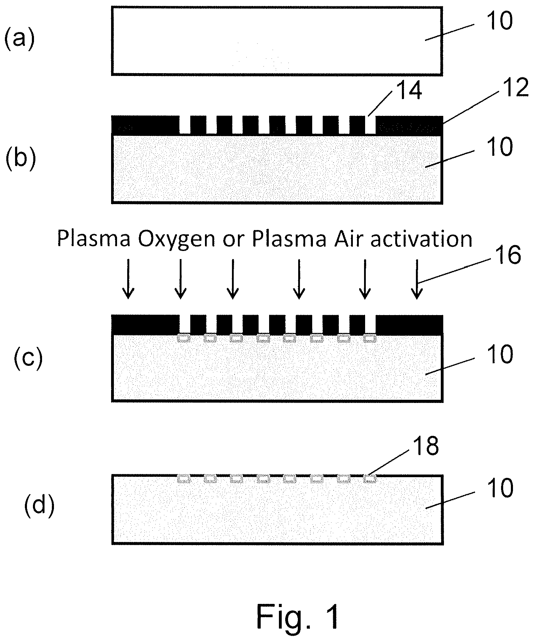

The second elements serve as spacers between the members of the plurality of first elements. Similar to the first elements, the second elements are sized to control cell adhesion, growth and differentiation. In an exemplary embodiment, a first member of the plurality of first elements is separated from a second member of the plurality of first elements by a member of the plurality of second elements by a distance of from about 2µm to about 6µm. See, **FIG. 1****.**

These and other objects, features, aspects and advantages of the present invention will become more apparent from the following detailed description of the present invention

### BRIEF DESCRIPTION OF THE DRAWINGS

**FIG. 1** (a), (b), (c), (d) are diagrams showing how an implant is selectively activated though a mask or stencil;
**FIG. 2** is a graph illustrating how the plasma activation can be optimized, showing also in details (a) and (b) the wettability of a nonactivated and an activated surface;
**FIG. 3** is a graph showing the effect of selective plasma surface activation on myofibroblast differentiation;
**FIG. 4** (a), (b), (c) and (d) are photographs showing the effect of selective plasma surface activation on myofibroblast differentiation in-vivo;
**FIG. 5** (a), (b), (c) and (d) are photographs showing the effect of selective plasma surface activation on collagen capsular formation *in-vivo*;
**FIG. 6** is a graph showing the effect of selective plasma surface activation in-vivo compared to clinical controls; and
**FIG. 7** is a sketch of the apparatus to activate surfaces with a selective pattern: the apparatus consists in a plasma machine for corona treatment, and a stencil to selectively activate micro-islets on the surface of the implant, wound dressing or desired final product.

### DETAILED DESCRIPTION OF THE EMBODIMENTS

### Introduction

The invention provides a new method of micro-topographic activation of surfaces by plasma technology, where cell-adhesive activated hydrophilic areas are alternated to cell repellent, non-adhesive zones. It is an object of the invention to provide a medical device with a cell-adhesive surface and a method to produce it. The surface of the invention is designed to retard, inhibit or prevent fibrotic growth on the device and to inhibit, or prevent fibrotic encapsulation of the device. The surface of the invention is readily prepared by an efficient process, which involves fewer steps than prior methods of preparing biocompatible surfaces.

Before the invention is described in greater detail, it is to be understood that the invention is not limited to particular embodiments described herein as such embodiments may vary. It is also to be understood that the terminology used herein is for the purpose of describing particular embodiments only, and the terminology is not intended to be limiting. The scope of the invention will be limited only by the appended claims. Unless defined otherwise, all technical and scientific terms used herein have the same meaning as commonly understood by one of ordinary skill in the art to which this invention belongs. Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention. Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number, which, in the context in which it is presented, provides the substantial equivalent of the specifically recited number.

As will be apparent to those of skill in the art upon reading this disclosure, each of the individual embodiments described and illustrated herein has discrete components and features which may be readily separated from or combined with the features of any of the other several embodiments without departing from the scope or spirit of the invention. Any recited method may be carried out in the order of events recited or in any other order that is logically possible. Although any methods and materials similar or equivalent to those described herein may also be used in the practice or testing of the invention, representative illustrative methods and materials are now described.

In describing the present invention, the following terms will be employed, and are defined as indicated below.

### Definitions

"Fibrosis", "scarring", or "fibrotic response" refers to the formation of fibrous tissue or any excess production, accumulation or deposition of extracellular matrix components (including collagen and other biomolecules) with or without cell infiltration, in response to injury or medical intervention which includes biomaterial implantation, and is referred to as "fibrosis" hereafter. An example of fibrosis is that induced by a foreign body response of a subject. Therapeutic agents that promote fibrosis, which may be called "fibrosis-inducing agents," "scarring agents," "adhesion-inducing agent," "fibrosing agent", and the like, promote fibrosis through one or more mechanisms including: inducing or promoting angiogenesis, stimulating migration or proliferation of soft tissue or connective tissue cells (such as fibroblasts, smooth muscle cells, and vascular smooth muscle cells), inducing ECM production, and/or promoting tissue remodeling. Correspondingly, exemplary "fibrosis inhibiting agents" act by interfering with one or more of these mechanisms.

"Foreign body response" is defined as a reaction of biological tissue to any foreign material in the tissue. "Fibrosis" is one kind of foreign body response. "Foreign body response" in the form of fibrosis is traditionally considered an undesirable "side effect" because it is triggered by an inflammatory response. As a result, past researchers have failed to appreciate any positive aspects of cell-infiltrated collagen formation by fibrosis.

"Implant", as noun, is used herein to include any material placed inside the body by any method other than ingestion or inhalation, or placed on the surface of an open wound. "Implant", as a verb, refers herein to the process of putting the material inside the body by any method other than ingestion or inhalation. The implant can be placed in or adjacent to any organ or tissue.

"Tissue" or "Organ" is used herein to refer to any natural or engineered biological tissue or organ, including, but not limited to, vascularized tissues and avascular tissues, including musculoskeletal tissue, such as cartilage, menisci, muscles, ligaments and tendons, skin, cardiovascular tissue, neuronal tissue, periodontal tissue, glandular tissue, organ tissue, islets of Langerhans, cornea, ureter, urethra, breast tissue, and organs, such as pancreas, bladder, kidney, breast, liver, intestine, heart and sections or pieces thereof.

"Internal organ" herein refers to any organ as defined above, but excluding skin.

"Soft tissue" refers to tissues that connect, support, or surround other structures and organs of the body. Soft tissue includes muscles, tendons (bands of fiber that connect muscles to bones), fibrous tissues, fat, blood vessels, nerves, stroma, ligaments and synovial tissues other than ligament. Specifically, as used herein, it does not include cartilage or skin.

"Membranous tissue" is used herein to mean any tissue of an animal that forms a sheet or sheath; membranous tissue commonly encloses or delimits a tissue, or divides an organ into separate compartments.

"Cardiovascular tissue" refers to any tissue of the cardiovascular system, and is exemplified to include blood vessels, capillaries, heart, myocardium, heart valves, blood vessel valves, and any membranous or non-membranous tissue of the cardiovascular system.

"Growth factor" refers to a substance that is effective to promote the growth of cells and which, unless added to the culture medium as a supplement, is not otherwise a component of the basal medium. Put another way, a growth factor is a molecule that is not secreted by cells being cultured (including any feeder cells, if present) or, if secreted by cells in the culture medium, is not secreted in an amount sufficient to achieve the result obtained by adding the growth factor exogenously. Growth factors include, but are not limited to, basic fibroblast growth factor (bFGF), acidic fibroblast growth factor (aFGF), epidermal growth factor (EGF), insulin-like growth factor-I (IGF-T), insulin-like growth factor-II (IGF-II), platelet-derived growth factor-AB (PDGF), vascular endothelial cell growth factor (VEGF), activin-A, bone morphogenic proteins (BMPs), insulin, cytokines, chemokines, morphogens, neutralizing antibodies, other proteins, and small molecules.

An "autologous cell", according to the present is a cell derived from a subject into whom a device of the invention will be implanted. Exemplary autologous cells include fibroblasts, adipose-derived cells, osteocytes, chondrocytes, myocytes, stem cells or blood-derived cells. In various embodiments, prior to implantation of the device, autologous cells are bound to the first surface (islets). In various embodiments, the body of the subject itself deposits autologous cells on the first surface of the invention following implantation.

Generally speaking, a "hydrophobic" surface is one that lacks an affinity, or has a low affinity, for polar liquid, such as those containing water; a "hydrophilic" surface is one that has an affinity for polarized solutions, such as those containing water. As used in the context of the present invention, a "hydrophilic" surface has a greater affinity for a particular polar liquid than a "hydrophobic" surface.

As used herein, "drugs" includes all types of therapeutic agents, whether small molecules or large molecules such as proteins, nucleic acids and the like. The drugs incorporated into the device of the invention can be used alone or in combination.

"Device", as used herein, refers, without limitation, to implantable or insertable medical devices, for example, stents (including coronary vascular stents, peripheral vascular stents, cerebral, urethral, ureteral, biliary, tracheal, gastrointestinal and esophageal stents), stent coverings, stent grafts, vascular grafts, abdominal aortic aneurysm (AAA) devices (e.g., AAA stents, AAA grafts), vascular access ports, dialysis ports, catheters (e.g., urological catheters or vascular catheters such as balloon catheters and various central venous catheters), guide wires, balloons, filters (e.g., vena cava filters and mesh filters for distil protection devices), embolization devices including cerebral aneurysm filler coils (including Guglilmi detachable coils and metal coils), septal defect closure devices, myocardial plugs, patches, pacemakers, pacemaker leads, defibrillation leads, and coils, neural stimulators including neural stimulation leads, ventricular assist devices including left ventricular assist hearts and pumps, total artificial hearts, shunts, valves including heart valves and vascular valves, anastomosis clips and rings, auditory (e.g., cochlear implants), tissue bulking devices, and tissue engineering scaffolds for cartilage, bone, skin and other *in vivo* tissue regeneration, sutures, suture anchors, tissue staples and ligating clips at surgical sites, cannulae, metal wire ligatures, urethral slings, hernia "meshes", artificial ligaments, orthopedic prosthesis such as bone grafts, bone plates, fins and fusion devices, joint prostheses, orthopedic fixation devices such as interference screws in the ankle, knee, and hand areas, tacks for ligament attachment and meniscal repair, rods and pins for fracture fixation, screws and plates for craniomaxillofacial repair, dental implants, ocular implants, breast implants or other devices that are implanted or inserted into the body.

As used herein, "cell-adhesive protein" includes, collagen (e.g., type II, etc,), fibronectin and RDG.

### The Device

The invention provides a surface with anti-fibrotic properties. In the devices of the invention, anti-fibrotic is defined as retarding, inhibiting or preventing the attachment of cells responsible for fibrotic tissue formation and contraction. For example, an anti-fibrotic surface of the invention retards, inhibits or prevents the attachment of myofibroblasts to the surface or the transformation of fibroblasts bound to the surface to myofibroblasts. In various embodiments, the anti-fibrotic surface of the invention has bio-physical properties allowing fibroblasts to attach, but impair myofibroblast development.

Myofibroblast development is readily determined by an *in vitro* assay in which fibroblasts are seeded on the surface and myofibroblast differentiation is induced with TGF-β1. Myofibroblasts are defined as cells positive for α-SMA. Thus, in an exemplary embodiment, the invention provides a surface as described herein, which, when tested for α-SMA provides a substantially negative read out.

In an exemplary embodiment, the invention provides a device configured for implantation or wound dressing in a subject. The device comprises a device surface. The device surface comprises a plurality of first elements. Each of the first elements has a first surface. The device surface also includes a plurality of second elements. Each second element comprises a second surface. Each first surface has a first affinity to water. In an exemplary embodiment, the first surface is hydrophilic, or is more hydrophilic than the second surface. Similarly, each second surface has an affinity for water. In various embodiments, the second surface is hydrophobic, or is more hydrophobic than the first surface. In general, the first affinity and the second affinity are different affinities.

In an exemplary embodiment, the surface is prepared by plasma treatment of a precursor device surface. More specifically, in various embodiments, the first surface is prepared by plasma treatment of said device surface. The first surface is an oxidized surface.

The elements on the device surface are sized to control cell adhesion, growth and differentiation. Thus, in an exemplary embodiment, each first element has a length of less than about 6µm and a width of less than about 2µm.

The second elements can be conceptualized as spacers between the members of the plurality of first elements. Similar to the first elements, the second elements are sized to control cell adhesion, growth and differentiation. In an exemplary embodiment, a first member of the plurality of first elements is separated from a second member of the plurality of first elements by a member of the plurality of second elements by a distance of from about 2µm to about 6µm. See, **FIG. 1****.**

The invention provides a treated surface of a device for implantation or for application as a wound dressing. The treated surface comprises a plurality of plasma-activated hydrophilic cell-adhesive areas having the ability to reduce fibrous reaction. The members of the plurality of plasma-activated hydrophilic cell-adhesive areas are separated by a plurality of hydrophobic non-cell-adhesive regions. In various embodiments, the islets of activation are hydrophilic and are surrounded by non-activated, non-adhesive, hydrophobic areas. In an exemplary embodiment, the geometry of the activated islets that best reduces fibrosis is characterized by an array of single islets, wherein the islets have a length that is less than or equal to about 6µm, a width that is less than or equal to about 2µm and distance between them that is from about 2µm to about 6 µm, e.g., from about 4µm to about 6 µm.

A material is cell-adhesive if a cell will form an adhesive contact with a surface of the material. A cell that has adhered to a material will not be removed from that surface by mechanical stress such as that associated with rinsing the material with water or a buffer solution. In an exemplary embodiment, the first elements (e.g., islets) of the surface are sufficiently cell-adhesive to provide the device with anti-fibrotic character without further augmentation with a cell-adhesion factor. In an exemplary embodiment, the second surface is not cell-adhesive itself. In various embodiments, the first element further comprises a cell-adhesion factor to promote cell adhesion.

In various embodiments, the first plurality of first elements and the plurality of second elements are arranged in a pattern and the pattern is antifibrotic.

A cell-adhesion factor is any chemical entity that promotes or mediates specific adhesion of a cell to another material, e.g., a surface of the invention. Such factors fibronectin and collagen.

Specific adhesion is adhesion that is mediated by reversible or irreversible bonds between specific, complementary molecules collagen and fibronectin. In the present invention, mechanisms that are known to promote specific adhesion among cells may be adapted to promote adhesion between cells and the surface of the invention by linking the complementary molecules to the surface of the invention. In an exemplary embodiment, the surface of the material provides a substrate for the specific adhesion of fibroblasts to the surface.

Specific adhesion is to be distinguished for the purposes of the invention from non-specific adhesion. Non-specific adhesion includes mechanisms of adhesion such as electrostatic attraction, hydrogen bonding, covalent bonding, and other mechanisms of adhesion that do not rely on the reversible bonding between complementary molecules. In an exemplary embodiment, the surface of the invention provides a substrate for the non-specific adhesion of fibroblasts to the surface.

In an exemplary embodiment, the first surface provides a substrate for specific adhesion of a cell, and the second surface does not provide a substrate for specific adhesion of a cell. In various embodiments, the cell is a fibroblast.

A differentially adhesive surface, with respect to cell adhesion, permits the adhesion of one type of cell to the surface, while resisting the adhesion of a different type of cell. Thus, in an exemplary embodiment, the surface of the invention provides a substrate for adhesion of fibroblasts to the surface. Myofibroblasts either do not adhere to the surface or adhere to the surface in a manner that allows fibroblasts to functionally predominate the adherent cellular population, thereby effectively inhibiting, retarding or preventing fibrosis.

In exemplary embodiments, the device surface is made of any useful material including, without limitation, metal, metalloid, polymer, organic material, ceramic, metal oxide, metalloid oxide, or a combination thereof. In an exemplary embodiment, the surface does not comprise glass.

In an exemplary embodiment, the device of the invention is at least partially formed from a synthetic polymer. Exemplary synthetic polymers include polyurethanes, silicones and polyesters. In various embodiments, the synthetic polymer is selected from polyhydroxyvalerate, poly(L)lactic acid, polycaprolactone, polylactide-co-glycolide, polyhydroxybutyrate, polyhydroxybutyrate-co-valerate, polydioxanone, polyorthoesters, polyanhydrides, polyacetic, polyglycolic acid, poly(D,L)lactic acid, polyglycolic acid-co-trimethylene carbonate, polysebacic acid, polylactic-co-sebacic acid, polyglycolic-co-sebacid acid, polyphosphoesters, polyphosphoester urethanes, polyamino acids, ion exchange resins, cyanoacrylates, polytrimethylene carbonate, polyiminocarbonate, copolyether-esters, polyalkylene oxalates and polyphosphazenes. Other synthetic polymers of use include polyolefin, polyisobutylene, ethylene-alphaolefin copolymers, acrylic polymers and copolymers, vinyl halide polymers and copolymers, polyvinyl ethers, polyvinylidene halides, polyacrylonitrile, polyvinyl ketones, polyvinyl aromatics, polyvinyl esters, copolymers of vinyl monomers with each other and olefins, polyamides, alkyd resins, polycarbonates, polyoxymethylenes, polyimides, polyethers, epoxy resins, rayon and rayon-triacetate.

In various embodiments, the surface is an activated surface with a water contact angle of from about 40° to about 60°. In various embodiments, the surface is an activated silicone surface with a water contact angle of from about 40° to about 60°.

Exemplary devices of the invention include implants and wound dressings, including, but not limited to, prostheses, such as joint replacements, artificial tendons and ligaments, dental implants, blood vessel prostheses, heart valves, cochlear replacements, intraocular lens, brain, central and peripheral nervous system implants, mammary prostheses, penile and testicular prostheses, and tracheal, laryngeal, and esophageal replacement devices; tissue expanders; artificial organs such as heart, liver, pancreas, kidney, and parathyroid; and repair materials and devices such as bone, and cartilage and orthopaedic implants, bone cements, bone defect repairs, bone plates for fracture fixation, heart valves, catheters, nerve regeneration channels, corneal bandages, skin repair templates, and scaffolds for tissue repair and regeneration, wound dressings, including dressings and wound interfaces connected to a vacuum (negative pressure dressings); and devices such as pacemakers, implantable drug delivery systems (e.g., for drugs, human growth hormone, insulin, bone growth factors, and other hormones), and biosensors. Implantable drug delivery systems are disclosed in U.S. Pat. Nos. 3,773,919, 4,155,992, 4,379,138, 4,130,639, 4,900,556, 4,186,189, 5,593,697, and 5,342,622. Biosensors for monitoring conditions such as blood pH, ion concentration, metabolite levels, clinical chemistry analyses, oxygen concentration, carbon dioxide concentration, pressure, and glucose levels are known. Blood glucose levels, for example, may be monitored using optical sensors and electrochemical sensors. Various UV, HPLC and protein activity assays are known or can be modified to provide quantitation of the release rates, concentration, and activity of the tissue response modifiers *in vitro* and *in vivo.*

The micro-geometry of activation of islets **18** according to the invention to best reduce fibrosis includes an array of islets, wherein the islets **18** have a length that is less than or equal to about 6µm, a width that is less than or equal to about 2µm and distance between them that is from about 2µm to about 6µm e.g., from about 4µm to about 6µm. The activated islets **18** can have any geometrical shape, providing they retain the specified size and distribution.

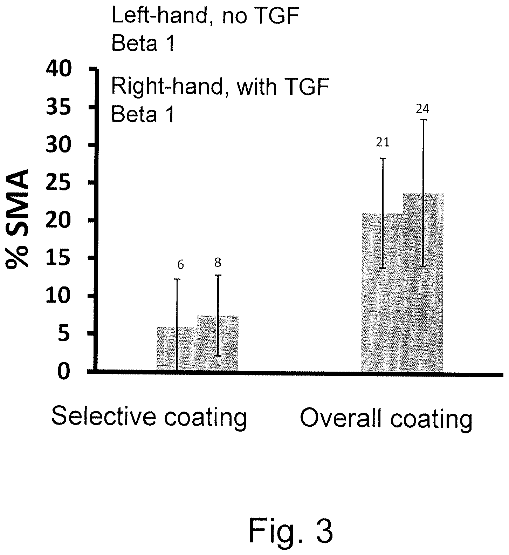

**FIG. 3** is a graph showing the effect of selective plasma surface activation on myofibroblast differentiation and illustrates that selectively activated islets are efficient in reducing the % of SMA in the samples, whereas samples activated over their entire surface shown a much higher % of SMA. Notably, *in vitro,* on silicone, selective activation of specific islet size (length of 4µm and width of 2µm) and distribution with a regular distance between the islets of 5µm reduced 4-fold the differentiation of human dermal fibroblasts to myofibroblasts compared to fully activated silicone surfaces or selectively activated substrates with islets of bigger size. The activation of islets of greater size allowed the differentiation of fibroblasts into myofibroblasts similarly to fully activated surfaces. These contractile cells were seen in high percentage (up to 24%) when the surface was coated globally compared to the specific islets provided by this invention where the contractile cells were about 5-7% (**FIG. 3**). The effect was independent from the presence of TGF-beta (**FIG. 3**).

***In vivo,*** PDMS substrates were implanted subcutaneously on the dorsum of rats. Two non-activated, one fully (non-selectively) activated (50W, 30s) and one activated (50W, 30s) with a micro-topography of islets with a length of 5µm, a width of 2µm and distance between them of 5µm (activated using a metallic *stenci*l) were implanted in 20 animals and followed up for one month.

Results show a similar presence of myofibroblasts at the interface of non-activated or fully activated PDMS surfaces as measured by Alpha-SMA positivity. Non-activated and fully activated implants induced 56% and 46% positivity of alpha-SMA, respectively, while selectively activated implants with islets of a length of 5µm, a width of 2µm and distance between them of 5µm reached 20% (**FIG. 3**). Selectively activated surfaces induced a 2.8 and 2.3 decrease in alpha SMA positivity (p<0.01) compared to non-activated and fully activated implants (**FIG. 3**).

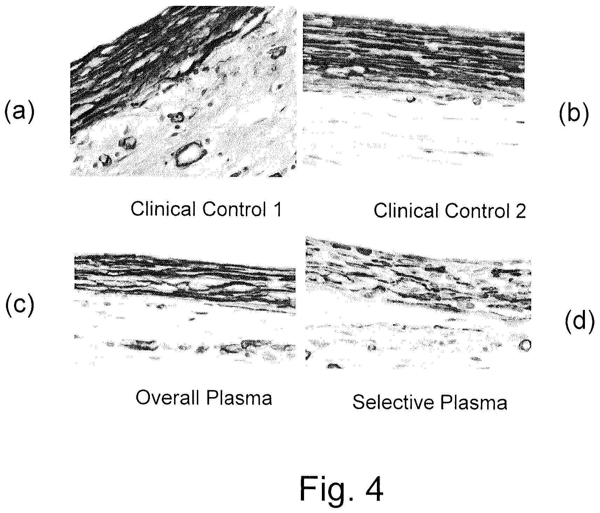

**FIG. 4** shows photographs where the interface with the implant is at the top an**d where** the dark striated part is myofibroblast. The lower interface **is the** int*erface* with the animal. As can be seen from **FIG. 4** (d) the inventive selective activation leads to less dense myofibroblasts than for overall plasma activation (**FIG. 4** (c)) or the clinical controls, **FIG. 4** (a), and **FIG. 4**(b).

The organization of the alpha-SMA fibers at the interface of fully activated and non-activated implants was characterized by parallel, densely packed and with low cellularity Alpha-SMA fibers (**FIG. 3**). Selectively activated implants with islets of a length of 4µm, a width of 2µm and distance between them of 5µm did not show this pattern of distribution of alpha-SMA exhibiting scattered and disorganized and less dense Alpha-SMA fibers (**FIG. 3**).

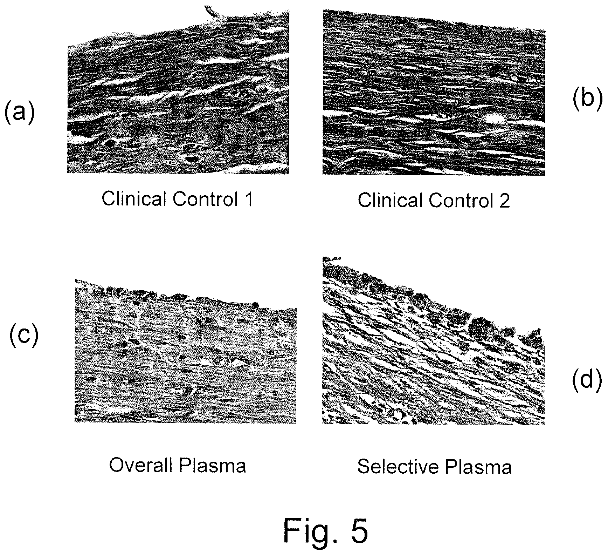

**FIG. 5** shows photographs in which the dark striated part is collagen. As shown in the photographs (a), (b), (c) and (d) of **FIG. 5** collagen deposition (scar forming) was also influenced by the coating: dense parallel fibers of collagen were deposited around non-activated (**FIG. 5** (a) and (b)) and fully activated implants (**FIG. 5** (c)). Implants with islets of activation a length of 4µm, a width of 2µm and distance between them of 5µm significantly reduced the deposition of collagen (**FIG. 5** (d)).

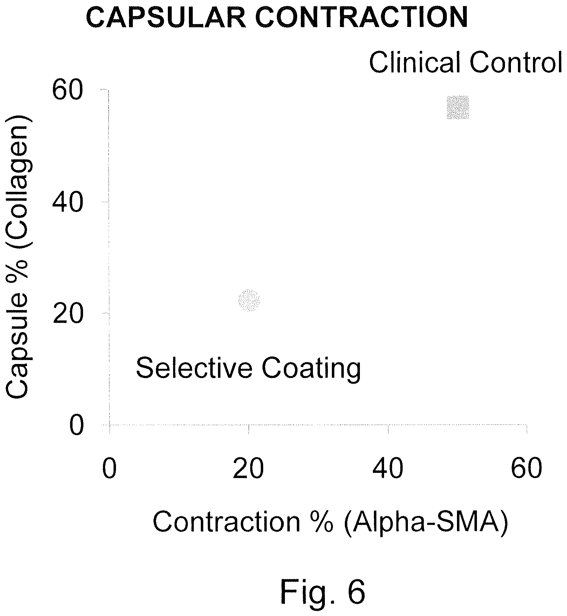

In summary, the selective activation pattern (islets of activation length 5µm, width 2µm and distance between them of 5µm) induced a significant decrease in myofibroblasts and capsule deposition compared to clinical controls - see, **FIG. 6** (d) compared to **FIG. 6** (a) and (b), non-activated, and **FIG. 5** (c), overall, non selectively activated.

The micro-geometry of activated islets for example with a length of 4µm, a width of 2µm and distance between them of 5µm can be applied to any medical device.

### Bioactive Substance

In an exemplary embodiment, the surface of the first element (islet) of the device is functionalized with a bioactive substance. In various embodiments, the bioactive substance is selected from agents promoting cellular adhesion, cells and therapeutic agents. In various embodiments, the bioactive substance retards or prevents development of fibrotic tissue on said first surface. In an exemplary embodiment, the second surface does not comprise a bioactive substance bound thereto. Any bioactive substance (e.g., drug, biological agent) compatible with the process of preparing the device of the invention can be incorporated into the device. Amounts of the bioactive substance to required to provide a desired effect are known in the art. Those of skill in the art can readily determine the amount of a particular bioactive substance to be incorporated in a device of the invention.

Examples of bioactive substances suitable for use with the present invention include a protein, e.g., collagen, fibronectin, and RDG, and a polypeptide, e.g., poly-N-acetylglucosamine, anti-inflammatory agents, anesthetics, antibiotics (antimicrobials), fibrosis-inhibiting agents, anti-scarring agents, leukotriene inhibitors/antagonists, cell growth factors, cell growth inhibitors and the like.

Examples of non-steroidal anti-inflammatories include, but are not limited to, naproxen, ketoprofen, ibuprofen as well as diclofenac; celecoxib; sulindac; diflunisal; piroxicam; indomethacin; etodolac; meloxicam; r-flurbiprofen; mefenamic; nabumetone; tolmetin, and sodium salts of each of the foregoing; ketorolac bromethamine; ketorolac bromethamine tromethamine; choline magnesium trisalicylate; rofecoxib; valdecoxib; lumiracoxib; etoricoxib; aspirin; salicylic acid and its sodium salt; salicylate esters of alpha, beta, gamma-tocopherols and tocotrienols (and all their D-, L-, and racemic isomers); and the methyl, ethyl, propyl, isopropyl, n-butyl, sec-butyl, t-butyl, esters of acetylsalicylic acid.

Examples of anesthetics include, but are not limited to, lidocaine, bupivacaine, and mepivacaine. Further examples of analgesics, anesthetics and narcotics include, but are not limited to acetaminophen, clonidine, benzodiazepine, the benzodiazepine antagonist flumazenil, lidocaine, tramadol, carbamazepine, meperidine, zaleplon, trimipramine maleate, buprenorphine, nalbuphine, pentazocain, fentanyl, propoxyphene, hydromorphone, methadone, morphine, levorphanol, and hydrocodone. Local anesthetics have weak antibacterial properties and can play a dual role in the prevention of acute pain and infection.

Examples of antimicrobials include, but are not limited to, triclosan, chlorhexidine, rifampin, minocycline (or other tetracycline derivatives), vancomycin, daptomycin, gentamycin, cephalosporins and the like. In exemplary embodiments the coatings contain rifampin and another antimicrobial agent, preferably that agent is a tetracycline derivative. In another exemplary embodiment, the device contains a cephalosporin and another antimicrobial agent. Exemplary combinations include rifampin and minocycline, rifampin and gentamycin, and rifampin and minocycline. As used herein, the term antibiotic and antibacterial can be used interchangeably with the term antimicrobial.

Further antimicrobials include aztreonam; cefotetan and its disodium salt; loracarbef; cefoxitin and its sodium salt; cefazolin and its sodium salt; cefaclor; ceftibuten and its sodium salt; ceftizoxime; ceftizoxime sodium salt; cefoperazone and its sodium salt; cefuroxime and its sodium salt; cefuroxime axetil; cefprozil; ceftazidime; cefotaxime and its sodium salt; cefadroxil; ceftazidime and its sodium salt; cephalexin; cefamandole nafate; cefepime and its hydrochloride, sulfate, and phosphate salt; cefdinir and its sodium salt; ceftriaxone and its sodium salt; cefixime and its sodium salt; cefpodoxime proxetil; meropenem and its sodium salt; imipenem and its sodium salt; cilastatin and its sodium salt; azithromycin; clarithromycin; dirithromycin; erythromycin and hydrochloride, sulfate, or phosphate salts ethylsuccinate, and stearate forms thereof; clindamycin; clindamycin hydrochloride, sulfate, or phosphate salt; lincomycin and hydrochloride, sulfate, or phosphate salt thereof; tobramycin and its hydrochloride, sulfate, or phosphate salt; streptomycin and its hydrochloride, sulfate, or phosphate salt; vancomycin and its hydrochloride, sulfate, or phosphate salt; neomycin and its hydrochloride, sulfate, or phosphate salt; acetyl sulfisoxazole; colistimethate and its sodium salt; quinupristin; dalfopristin; amoxicillin; ampicillin and its sodium salt; clavulanic acid and its sodium or potassium salt; penicillin G; penicillin G benzathine, or procaine salt; penicillin G sodium or potassium salt; carbenicillin and its disodium or indanyl disodium salt; piperacillin and its sodium salt; ticarcillin and its disodium salt; sulbactam and its sodium salt; moxifloxacin; ciprofloxacin; ofloxacin; levofloxacins; norfloxacin; gatifloxacin; trovafloxacin mesylate; alatrofloxacin mesylate; trimethoprim; sulfamethoxazole; demeclocycline and its hydrochloride, sulfate, or phosphate salt; doxycycline and its hydrochloride, sulfate, or phosphate salt; minocycline and its hydrochloride, sulfate, or phosphate salt; tetracycline and its hydrochloride, sulfate, or phosphate salt; oxytetracycline and its hydrochloride, sulfate, or phosphate salt; chlortetracycline and its hydrochloride, sulfate, or phosphate salt; metronidazole; dapsone; atovaquone; rifabutin; linezolide; polymyxin B and its hydrochloride, sulfate, or phosphate salt; sulfacetamide and its sodium salt; and clarithromycin.

Examples of antifungals include amphotericin B; pyrimethamine; flucytosine; caspofungin acetate; fluconazole; griseofulvin; terbinafin and its hydrochloride, sulfate, or phosphate salt; ketoconazole; micronazole; clotrimazole; econazole; ciclopirox; naftifine; and itraconazole.

Other drugs that can be incorporated into the devices of the invention include, but are not limited to, keflex, acyclovir, cephradine, malphalen, procaine, ephedrine, adriamycin, daunomycin, plumbagin, atropine, quinine, digoxin, quinidine, biologically active peptides, cephradine, cephalothin, cis-hydroxy-L-proline, melphalan, penicillin V, aspirin, nicotinic acid, chemodeoxycholic acid, chlorambucil, paclitaxel, sirolimus, cyclosporins, 5-fluorouracil and the like.

Examples of anti-inflammatory compound include, but are not limited to, anecortive acetate; tetrahydrocortisol, 4,9(11)-pregnadien-17.alpha., 21-diol-3,20-dione and its -21-acetate salt; 11-epicortisol; 17α-hydroxyprogesterone; tetrahydrocortexolone; cortisona; cortisone acetate; hydrocortisone; hydrocortisone acetate; fludrocortisone; fludrocortisone acetate; fludrocortisone phosphate; prednisone; prednisolone; prednisolone sodium phosphate; methylprednisolone; methylprednisolone acetate; methylprednisolone, sodium succinate; triamcinolone; triamcinolone-16,21-diacetate; triamcinolone acetonide and its -21-acetate, -21-disodium phosphate, and -21-hemisuccinate forms; triamcinolone benetonide; triamcinolone hexacetonide; fluocinolone and fluocinolone acetate; dexamethasone and its - 21-acetate, -21-(3,3-dimethylbutyrate), -21-phosphate disodium salt, -21-diethylaminoacetate, -21-isonicotinate, -21-dipropionate, and -21-palmitate forms; betamethasone and its -21-acetate, -21-adamantoate, -17-benzoate, -17,21-dipropionate, -17-valerate, and -21-phosphate disodium salts; beclomethasone; beclomethasone dipropionate; diflorasone; diflorasone diacetate; mometasone furoate; and acetazolamide.

Examples of leukotriene inhibitors/antagonists include, but are not limited to, leukotriene receptor antagonists such as acitazanolast, iralukast, montelukast, pranlukast, verlukast, zafirlukast, and zileuton.

Another useful drug that can be incorporated into the coatings of the invention is sodium 2-mercaptoethane sulfonate (Mesna). Mesna has been shown to diminish myofibroblast formation in animal studies of capsular contracture with breast implants (Ajmal et al. (2003) Plast. Reconstr. Surg. 112:1455-1461).

Any pharmaceutically acceptable form of the drugs used in the device of the present invention can be employed in the present invention, e.g., the free base or a pharmaceutically acceptable salt or ester thereof. Pharmaceutically acceptable salts, for instance, include sulfate, lactate, acetate, stearate, hydrochloride, tartrate, maleate, citrate, phosphate and the like.

In various embodiments, the first surface comprises cells bound thereto, and the cells are autologous cells from said subject. Exemplary autologous cells include fibroblasts, adipose-derived cells, osteocytes, chrondrocytes, myocytes, stem cells, blood-derived cells and a combination thereof.

In various embodiments, the second surface does not comprise cells bound thereto.

Those of ordinary skill in the art will appreciate that any of the foregoing disclosed drugs can be used in combination or mixture in coatings of the present invention.

### Methods of Making

In exemplary embodiments, the invention provides a method of treating a surface of a device (or a material that will be incorporated into a device as its surface) for implantation or application as a wound dressing. The method includes transforming regions of a hydrophobic surface into hydrophilic "elements" or "islets". The method for converting regions of the hydrophobic surface to hydrophilic regions includes oxidizing those regions that are to be converted to hydrophilic regions. An exemplary method of oxidizing the desired regions is to expose them to a plasma, though, as will be appreciated by those of skill in the art, other methods are available and applicable.

The invention also provides a method of treating the surface of a device for implantation or for application as a wound dressing, the method comprising treating the surface by localized plasma activation to produce a plurality of plasma-activated hydrophilic cell-adhesive areas having the ability to reduce fibrous reaction, said plurality of plasma-activated hydrophilic cell-adhesive areas being produced in the form of an array of islets of activation, each islet of activation having a length which is less than or equal to about 6µm, a width which is less than or equal to about 2µm and wherein the distance between islets is from about 2 µm to about 6 µm, e.g., from about 4µm to about 6µm, the islets of activation being surrounded by non-activated, non-adhesive, hydrophobic areas.

According to one embodiment of the invention the selective plasma activation is applied to the medical device surface by a stencil or mask, i.e., a template with holes of the size and distribution of the islets.

For selectively activating the surface according to another embodiment, the micro-stencil or mask can be brought in contact with the surface of the medical device and the activation of the surface happens through the holes of the stencil.

According to another embodiment, the stencil or mask can be attached to the plasma machine and brought in contact to the medical implant to selectively activate the islets.

**FIG. 1**(a) schematically shows a medical device that could be an implant **10,** for instance a mass of silicone. As shown in **FIG. 1**(b) a stencil or mask **12** having a series of through-openings **14** as applied to a surface of the implant **10.** These openings **14** are in an array that corresponds with the locations where the implant's surface is to be activated. Plasma oxygen or plasma air activation is then applied as indicated at **16** in **Fig. 1**(c) and this creates an array of islets of activation **18** on the implant surface, as shown in **FIG. 1**(d). These islets of activation **18** have a nanometric or molecular thickness on the activated surface, compared to a thickness of the order of a micrometer for proteins deposited according to WO 2010/026557. The activation involves few steps and the entire processing of an implant with the inventive process can take less than 2 minutes, compared to hours for the known multi-step deposition process of WO 2010/026557.

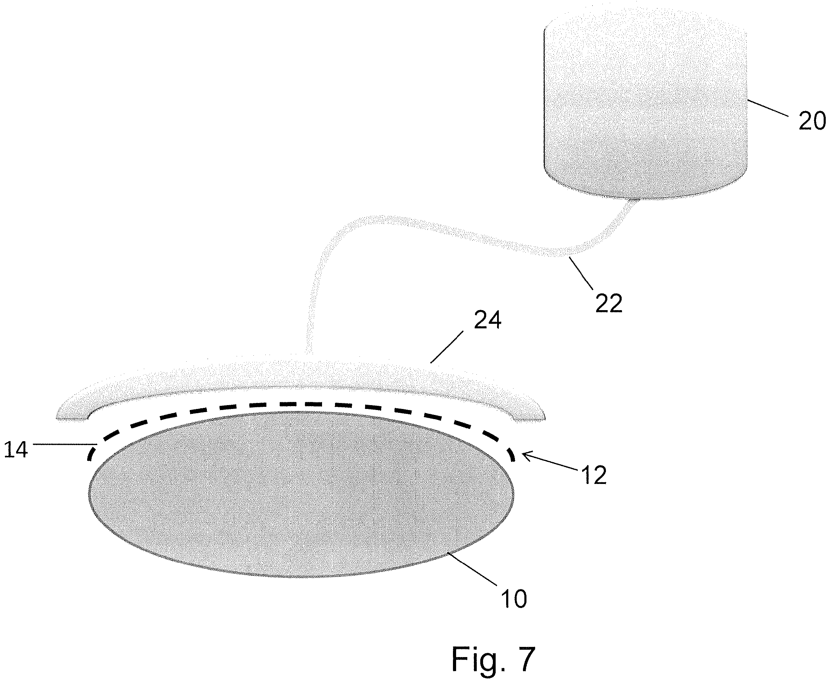

**FIG. 7** schematically shows an apparatus for carrying out the invention. As shown in this example, a plasma generator **20** as previously described is connected by a flexible tube **22** to a mobile applicator or head **24** configured to apply the plasma via the openings **14** in a stencil **12** applied on an implant **10** whose surface is to be treated. The tube **22** and head **24** form a plasma delivery system for local and specific application. The stencil **12** can be fitted to the head **24** and the two brought together against the implant. Or the stencil **12** can be applied on the implant **10** and the head **24** placed on top.

**The mic**ro-geometric selective activation guides cell adhesion on medical devices with the objective to reduce the fibro**ti**c reaction of the cells and tissues in contact with the device.

The mask or a stencil **12** to **a**ctivate the islets is micro fabricated using reco**gn**ized technolog**y**, e.g., photolithography, dry and wet etching, LIGA and laser cutting. Examples **of** a useful mask or stencil **12,** include soft stencils made from silicone, photo-resist and f**le**xible polymers and hard ste**nc**ils made from silicon, hard polymer and metal.

To micro-geometrical**ly** activate the surface of a medi**ca**l device **10** th**e** stencil **12** is first brought in conformal contact with the surface and then the plasma treatment starts. After plasma activation, the stencil **12** is removed and the anti-fibrotic micro-geometry remains on the implant surface (**FIG. 1** (d)).

The present invention provides a patterned, modified surface, which, in exemplary embodiments is anti-fibrotic. Exemplary methods of preparing the surfaces of the invention are generally recognized in the art and include plasma, X-ray, UV, laser, ion beam and e-beam etching, or corona discharge, which involves bombarding the surface with highly excited atomic, molecular, ionic, electronic, or free radical species to form reactive groups on an inert surface.

In one embodiment of the present invention, a plasma treatment technique is used to activate (e.g., oxidize, make hydrophilic) the surface of the first element. By way of a non-limiting example, one type of plasma treatment technique that can be employed in the practice of the present invention is electron cyclotron resonance (ECR), which allows a surface to be modified via exposure to a spatially localized gaseous plasma.

In general, an ECR plasma can be generated by providing a static magnetic field having a selected strength, i.e., amplitude, within a region of space in which a quantity of gas is contained, or through which the gas is flowing. The gas is then irradiated with electromagnetic radiation having a frequency which is substantially equal to ECR frequency at the applied magnetic field strength, and causes the gas to ionize, thus producing a plasma. The surface to be treated is exposed to a plasma for a time period ranging from about one second to about one minute. While different exposure times can be selected for different modifications of the surface, for example, shorter exposure times, such as one second, can be sufficient to activate the surface of the first element.

Any combination of the radiation frequency and magnetic field amplitude can be utilized. However, while various radiation frequencies and magnetic field strengths can be utilized to create and ECR-generated plasma, in an exemplary embodiment, the radiation frequency is selected to be in a range of about 1 GHz to about 15 GHz, and the applied static magnetic field is selected to have an amplitude in a range of approximately 300 Gauss to approximately 5500 Gauss.

Additionally, a variety of gasses and gas pressures can be used in conjunction with the magnetic field when forming an ECR plasma. These gases include, but are not limited to, noble gases, such as argon, diatomic gases, such as oxygen and nitrogen, hydrocarbons, such as methane and butane, and fluorinated hydrocarbons, such as tetrafluoromethane. Moreover, various mixtures of different gases can be utilized to create an ECR plasma in accordance with the teachings of the invention. For example, air, a mixture of argon and oxygen (e.g., a mixture having 50% molar concentration of argon and 50% molar concentration of oxygen) or a mixture of argon and ammonia can be used. Additionally, the gas pressure can be in a range of about 0.1 Pa to about 1000 Pa, e.g., about 1 Pa to about 10 Pa, e.g., about 2 Pa to about 8 Pa.

In an exemplary embodiment of the present invention, ion treatment can be used to activate the surface of the first element. The term "ion treatment" and similar wording as used herein is intended to encompass ion implantations, ion depositions, ion-beam-assisted deposition and ion-enhanced sputtering. As used in the present invention, ion treatment refers to any treatment of a surface of a first element (called a "localized area") by utilizing energized ions. For example, an ion-beam-assisted deposition (IBAD) process can be employed in which an ion source can accelerate ions into selected portions of a substrate for implantation therein. See, e.g., U.S. Pat. No. 5,520,664, for further details regarding the IBAD process and apparatus therefor.

The implanted ions can modify one or more surface properties of the of the first element to modulate, e.g., enhance, their affinity for functionalization relative to an element surface (e.g., a second element) not treated with ions. Alternatively, activation of the first element can occur by an ion implantation technique in which a selected number of localized areas (e.g., first element) are formed on a substrate surface by implanting one ion type in certain discrete regions of the substrate while other localized areas (e.g., second element) are formed on the substrate surface by implanting another ion type in other discrete regions. This results in a device surface having two types of localized elements, such that various localized elements have different surface properties relative to one another and/or relative to the remainder of the device surface.

The surface of the device can be activated by oxidation. While a variety of oxidation techniques can be used, the oxidation technique should preferably activate the surface of the first elements. By way of a non-limiting example, the oxidation technique can include exposing the semiconductor nanostructures to an oxidizing atmosphere at an elevated temperature. While the oxidizing atmosphere can have a variety of compositions, in an exemplary embodiment, it contains at least about 1% O₂. Alternatively, the oxidizing technique can include contacting the surface of the device with an oxidizing solution for a length of time such that oxidation occurs. While the oxidizing solution can have a variety of compositions, an exemplary oxidizing solution contains at least a percentage of sodium peroxide, nitric acid or sulfurous acid. Additionally, the length of the contact time can vary in accordance with the properties of the oxidizing solution, for example and as shown by U.S. Pat. No. 6,649,138, approximately 45 minutes to 1 hour is a suitable time frame for immersion in an H₂O₂ oxidizing solution so that the surface is oxidized at one monolayer thick.

In general, surface activation results in a desired modification of the first element's, and therefore the device's, surface properties. One such modification can be a change in hydrophilicity or hydrophobicity, e.g., render the first element hydrophilic. The term "hydrophilic" and its derivatives are used herein to describe materials that have an affinity for water and/or are capable of being dissolved or dispersed in water. One measure of a hydrophilic material is its ability to transfer from a non-aqueous to an aqueous phase in a dual phase system. For example, a "hydrophilic" compound typically will transfer from an organic phase to an aqueous phase, specifically from an organic, water-immiscible nonpolar solvent (e.g., with a dielectric constant less than about 5) to water, with a partition coefficient or greater than about 50%.

In various embodiments, the device surface is partitioned into activated first elements and inactive second elements in a desired pattern by the use of a mask disposed on the device surface. The mask permits selective treatment of the substrate surface, for example by an ion beam. A variety of masks can be utilized to selectively expose different portions of a device surface to plasma or ions. For example, the mask can be formed of silicon dioxide (SiO₂). The mask can be deposited on a silicon substrate, for example, by utilizing chemical vapor deposition (CVD) to deposit a masking layer that can be patterned by employing a number of known methods, such as photolithography. The patterned mask can provide a plurality of exposed and unexposed portion. The device surface can then be exposed to a plasma or beam of ions, such as nitrogen ions, having a selected energy based on a particular application (e.g., an ion energy in a range of about 0.1 keV to about 1000 keV) so as to impart a desired degree of activation to the exposed portions (e.g., first element). A person skilled in the art will appreciate the variety of ion implantation systems that can be employed for activating the surface of the device.

Array stencils can be micro-fabricated from parylene C, silicon wafer or metal (Nickel) with the techniques of photolithography, dry etching and metallization allowing for required resolution and good reproducibility of hole distribution on the stencils.

For the generation of the micro-geometric array of activated islets **18** the stencil was used during plasma activation over a period of typically 30 seconds.

The hydrophilicity of activated surfaces can be characterized by art-recognized techniques such as contact angle measurements. For example, the contact angle of a surface of the invention was determined with 1 microliter sessile water droplet. A contact angle between 40-60° was measured at the surface of silicone surface activated by plasma oxygen (50 w, 30 s). A silicone surface with this contact angle promoted cell adhesion. In an exemplary embodiment, the surface of the invention has a water contact angle for from about 40° to about 60°. In various embodiments, the surface is an activated silicone surface with a water contact angle of from about 40° to about 60°.

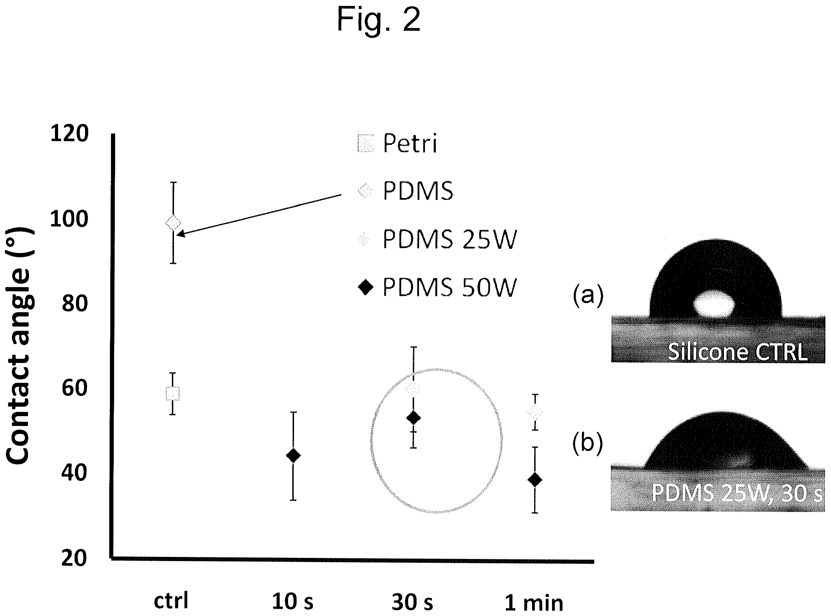

In contrast, a non-treated silicone surface had a contact angle between 90-110°. The results of these measurements are shown in **FIG. 2****,** using a Petri dish as control. The Petri dish had a contact angle of about 60 degrees, whereas a PDMS control had a contact angle of about 100 degrees. The measurements for PDMS activated at 25W and 50W for periods of 10 seconds, 30 seconds and 1 minute shows that the PDMS sample activated at 25W for 30 seconds shows an angle of contact of 60 degrees, identical to the Petri dish sample, and which is taken as ideal. **FIG. 2** (a) and (b) respectively show the water drop on the silicone control with a contact angle of 100 degrees, and the water drop on the PDMS sample activated at 25W for 30 seconds, with its angle of contact about 60 degrees.

Patterning efficiency (i.e., as a function of the cell attachment, or how many cells are attached to the substrate), as measured by cell density after 3 to 5 days of culture, was up to 4-5 times the density on non coated silicone, while being 2.5 times lower compared to fully activated surfaces, i.e., surfaces completely activated by the plasma, not selectively with islets.

In various embodiments, the invention provides an apparatus for patterning a surface of the invention (**FIG. 7**). In various embodiments, the apparatus is a portable apparatus, which consists of an air plasma machine for corona treatment. According to the invention, such apparatus comprises an air or oxygen plasma source, and a stencil or mask having an array of openings in correspondence to said islets of activation through which the plasma activation is to be applied to the treated surface, the stencil or mask being movable into contact with a surface of the device to be treated for the selective activation of the surface by the plasma source. The device can be in the form of or similar to the standard corona system or plasma, or in a pen-like, gun-like grippable, portable device, which can be directly applied on the surface of the implant or wound dressing. The corona surface treatment uses an electrode that generates high voltage discharge in air at high frequencies, between 10-30 kHz. The electrons in the air ionize the gas under this high voltage and attack the surface with enough force and energy to break the molecular bonds at the surface of the implant resulting in reactive free radicals. The selective activation of areas in the microscale range is possible, e.g., through a stencil, which is applied either on the implant or on the device.

The following non-limiting examples are offered to illustrate the invention.

### EXAMPLES

The invention describes a surface with anti-fibrotic properties. Anti-fibrotic is defined as having characteristics reducing the presence and development of myofibroblasts, cells responsible for fibrotic tissue formation and contraction. The bio-physical properties of the surface are sufficient to allow fibroblasts to attach, but impair myofibroblasts development.

Myofibroblast development is defined by an in vitro assay in which fibroblasts are seeded on the surface and myofibroblast differentiation is induced with TGF-β1. Myofibroblasts are defined as cells positive for α-SMA.

To identify the ideal properties of the antifibrotic properties several surface activation patterns were tested by the above mentioned assay. On silicone, selective activation of specific islet size (length of 4µm and width of 2µm) and distribution with a regular distance between the islets of 5µm reduced 4-fold the differentiation of human dermal fibroblasts to myofibroblasts compared to fully activated silicone surfaces (6% compared to 24% respectively) or selectively activated substrates with islets of bigger size.

In order to achieve anti fibrotic activation of the surface we modified its biochemical properties by rendering it hydrophilic. Ideal hydrophilicity is defined by a contact angle of around between 60 [40° and 80°], and specially 60° which was obtained with plasma oxygen settreatment at 25W for 30 seconds on PDMS silicone surface obtaining an angle of contact of 60 degrees, identical to the Petri dish samples, and which is taken as ideal. For other substrates (i.e., titanium) the goal is also to obtain selected hydrophilic zones with contact angle approximating the one of Petri dishes, which may be achieved with different plasma energy treatments or strong acids (or oxygen or Gas or air or corona plasma, or by strong acids, or by adding hydrophilic molecule) and length settings.

The selective activation of the surface may be achieved with a mask or stencil with the required resolution, which is brought in conformational contact to the surface. The stencil can be obtained by photolithography, dry and wet etching, LIGA and laser cutting, from silicone elastomer, parylene C, silicon wafer or metal (Nickel) material and several others.

## Claims

1. A treated surface of a device for implantation or for application as a wound dressing, the treated surface comprising a plurality of surface-oxidised in particular plasma-activated hydrophilic cell-adhesive areas having the ability to reduce fibrous reaction, said plurality of surface-oxidised in particular plasma-activated hydrophilic cell-adhesive areas being in the form of an array of islets of activation, each islet of activation having a length which is less than 6µm, a width which is less than 2 µm and wherein the distance between islets is from 2 µm to 6 µm, the islets of activation being surrounded by non-activated, non-adhesive, hydrophobic areas.

2. A treated surface according to claim 1 wherein the distance between islets is from 4 µm to 6 µm.

3. A treated surface according to claim 1 or 2 wherein the surface-oxidised in particular plasma activated hydrophilic cell-adhesive areas are covered with cell-adhesive proteins.

4. A treated surface according to claim 3 wherein the cell-adhesive proteins are selected from collagen, fibronectin and RDG.

5. A treated surface according to claim 1 or 2, wherein the surface-oxidised in particular plasma activated hydrophilic cell-adhesive areas are covered with polymers including for example poly-N-acetylglucosamine.

6. A treated surface according to any preceding claim wherein the surface-oxidised in particular plasma activated hydrophilic cell-adhesive areas of the treated surface, but not the non-activated areas, are covered with autologous cells from a subject to which the treated surface is to be implanted or applied as a wound dressing.

7. A treated surface according to claim 6 wherein the autologous cells include fibroblasts, adipose-derived cells, osteocytes, chondrocytes, myocytes, stem cells or blood-derived cells.

8. A device for implantation or for application as a wound dressing comprising a treated surface according to any preceding claims.

9. A method of treating the surface of a device for implantation or for application as a wound dressing, the method comprising treating the surface by localised oxidation treatment in particular plasma activation to produce a plurality of surface-oxidised in particular plasma-activated hydrophilic cell-adhesive areas having the ability to reduce fibrous reaction, said plurality of surface-oxidised in particular plasma-activated hydrophilic cell-adhesive areas being produced in the form of an array of islets of activation, each islet of activation having a length which is less than 6µm, a width which is less than 2 µm and wherein the distance between islets is from 2 µm to 6 µm, the islets of activation being surrounded by non-activated, non-adhesive, hydrophobic areas.

10. The method of claim 9 wherein the distance between islets is from 4 µm to 6 µm.

11. The method of claim 9 or 10, wherein a localized plasma activation to produce a plurality of plasma-activated hydrophilic cell-adhesive areas having the ability to reduce fibrous reaction is carried out using a stencil or mask having an array of openings in correspondence to said islets of activation through which openings the plasma activation is applied to the treated surface.

12. The method of claim 11 wherein the stencil or mask is brought in contact with the surface of the device to be treated for the activation.

13. The method of claim any one of claims 9 to 12, wherein prior to implantation of the device the surface-oxidised in particular plasma activated hydrophilic cell-adhesive areas, but not the non-activated areas, are covered with autologous cells from a subject to which the treated surface is to be implanted.

14. The method of claim 13 wherein the applied autologous cells include fibroblasts, adipose-derived cells, osteocytes, chondrocytes, myocytes, stem cells or blood-derived cell.

## Patentansprüche

1. Behandelte Oberfläche einer Vorrichtung zur Implantation oder zur Verwendung als Wundauflage, mit einer Vielzahl von oberflächenoxidierten, insbesondere plasma-aktivierten hydrophilen zelladhäsiven Bereichen mit der Fähigkeit, faserige Reaktion zu reduzieren, wobei die Vielzahl von oberflächenoxidierten, insbesondere plasma-aktivierten hydrophilen zelladhäsiven Bereichen die Form einer Anordnung von Aktivierungsinseln haben, jede mit einer Länge von weniger als 6 µm, einer Breite von weniger als 2 µm und mit einer Distanz zwischen den Inseln von 2 µm bis 6 µm, wobei die Aktivierungsinseln von nicht aktivierten, nichthaftenden hydrophoben Bereichen umgeben sind.

2. Behandelte Oberfläche nach Anspruch 1, wobei die Distanz zwischen den Inseln 4 µm bis 6 µm beträgt.

3. Behandelte Oberfläche nach Anspruch 1 oder 2, wobei die oberflächenoxidierten, insbesondere plasma-aktivierten hydrophilen zelladhäsiven Bereiche mit zelladhäsiven Proteinen bedeckt sind.

4. Behandelte Oberfläche nach Anspruch 3, wobei die zelladhäsiven Proteine aus Kollagen, Fibronectin und RDG ausgewählt sind.

5. Behandelte Oberfläche nach Anspruch 1 oder 2, wobei die oberflächenoxidierten, insbesondere plasma-aktivierten hydrophilen zelladhäsiven Bereiche mit Polymeren bedeckt sind, die zum Beispiel Poly-N-Acetylglucosamin umfassen.

6. Behandelte Oberfläche nach einem beliebigen der vorhergehenden Ansprüche, wobei die oberflächenoxidierten, insbesondere plasma-aktivierte, hydrophile, zelladhäsive, Bereiche der behandelten Oberfläche, aber nicht die nicht aktivierten Bereiche, mit autologen Zellen eines Patienten bedeckt sind, bei dem die behandelte Oberfläche implantiert oder als Wundauflage aufgebracht werden soll.

7. Behandelte Oberfläche nach Anspruch 6 wobei die autologen Zellen Fibroblasten, aus Fettgewebe gewonnene Zellen, Osteozyten, Chondrozyten, Myozyten, Stammzellen oder aus Blut gewonnene Zellen umfassen.

8. Vorrichtung zur Implantation oder zur Verwendung als Wundauflage mit einer behandelten Oberfläche nach einem beliebigen der vorhergehenden Ansprüche.

9. Verfahren zur Behandlung der Oberfläche einer Vorrichtung zur Implantation oder zur Verwendung als Wundauflage, wobei das Verfahren die Behandlung der Oberfläche durch lokalisierte Oxidationsbehandlung umfasst, insbesondere Plasmaaktivierung, um eine Vielzahl von oberflächenoxidierten, insbesondere plasma-aktivierten hydrophilen zelladhäsiven Bereichen mit der Fähigkeit, faserige Reaktion zu reduzieren, herzustellen, wobei die Vielzahl von oberflächenoxidierten, insbesondere plasma-aktivierten hydrophilen zelladhäsiven Bereichen die Form einer Anordnung von Aktivierungsinseln haben, jede mit einer Länge von weniger als 6 µm, einer Breite von weniger als 2 µm und mit einer Distanz zwischen den Inseln von 2 µm bis 6 µm, wobei die Aktivierungsinseln von nicht aktivierten, nichthaftenden hydrophoben Bereichen umgeben sind.

10. Verfahren nach Anspruch 9, wobei die Distanz zwischen den Inseln 4 µm bis 6 µm beträgt.

11. Verfahren nach Anspruch 9 oder 10, wobei eine lokalisierte Plasmaaktivierung, um eine Vielzahl von plasma-aktivierten hydrophilen zelladhäsiven Bereichen mit der Fähigkeit, faserige Reaktion zu reduzieren, unter Verwendung einer Schablone oder Maske durchgeführt wird, die eine Anordnung von Öffnungen an den Aktivierungsinseln aufweist, durch die hindurch die Plasmaaktivierung auf die behandelte Oberfläche aufgebracht wird.

12. Verfahren nach Anspruch 11, wobei die Schablone oder Maske für die Aktivierung in Kontakt mit der Oberfläche der zu behandelnden Vorrichtung gebracht wird.

13. Verfahren nach einem beliebigen der Ansprüche 9 bis 12, wobei vor der Implantation der Vorrichtung oder der Verwendung als Wundauflage die oberflächenoxidierten, insbesondere plasma-aktivierte, hydrophile, zelladhäsive, Bereiche, aber nicht die nicht aktivierten Bereiche, mit autologen Zellen eines Patienten bedeckt sind, in den die behandelte Oberfläche implantiert werden soll.

14. Verfahren nach Anspruch 13, wobei die aufgebrachten autologen Zellen Fibroblasten, aus Fettgewebe gewonnene Zellen, Osteozyten, Chondrozyten, Myozyten, Stammzellen oder aus Blut gewonnene Zellen umfassen.

## Revendications

1. Surface traitée d'un dispositif pour implantation ou pour application comme un pansement pour blessure, la surface traitée comprenant une pluralité de zones d'adhérence cellulaire hydrophiles à surface oxydée en particulier activée par plasma ayant la capacité de réduire la réaction fibreuse, ladite pluralité de zones d'adhérence cellulaire hydrophiles à surface oxydée en particulier activée par plasma ayant la forme d'un réseau d'îlots d'activation, chaque îlot d'activation ayant une longueur qui est inférieure à 6 µm, une largeur qui est inférieure à 2 µm et où la distance entre îlots est de 2 µm à 6 µm, les îlots d'activation étant entourés de zones hydrophobes non-activées, non-adhésives.

2. Surface traitée selon la revendication 1 où la distance entre îlots est de 4 µm à 6 µm.

3. Surface traitée selon la revendication 1 ou 2 où les zones d'adhérence cellulaire hydrophiles à surface oxydée en particulier activée par plasma sont couvertes de protéines d'adhérence cellulaire.

4. Surface traitée selon la revendication 3 où les protéines d'adhérence cellulaire sont sélectionnées parmi collagène, fibronectine et RGD.

5. Surface traitée selon la revendication 1 ou 2, où les zones d'adhérence cellulaire hydrophiles à surface oxydée en particulier activée par plasma sont couvertes de polymères comprenant par exemple poly-N-acétyl-glucosamine.

6. Surface traitée selon l'une quelconque des revendications précédentes où les zones d'adhérence cellulaire hydrophiles à surface oxydée en particulier activée par plasma de la surface traitée, mais non pas les zones non-activées, sont couvertes de cellules autologues d'un sujet auquel la surface traitée doit être implantée ou appliquée comme un pansement pour blessure.

7. Surface traitée selon la revendication 6 où les cellules autologues incluent fibroblastes, cellules d'origine adipeuse, ostéocytes, chondrocytes, myocytes, cellules souches ou cellules dérivées du sang.

8. Dispositif pour implantation ou pour application comme un pansement pour blessure comprenant une surface traitée selon l'une quelconque des revendications précédentes.

9. Méthode de traitement de la surface d'un dispositif pour implantation ou pour application comme un pansement pour blessure, la méthode comprenant le traitement de la surface par traitement par oxydation localisée en particulier activation par plasma pour produire une pluralité de zones d'adhérence cellulaire hydrophiles à surface oxydée en particulier activée par plasma ayant la capacité de réduire la réaction fibreuse, ladite pluralité de zones d'adhérence cellulaire hydrophiles à surface oxydée en particulier activée par plasma étant produite sous la forme d'un réseau d'îlots d'activation, chaque îlot d'activation ayant une longueur qui est inférieure à µm, une largeur qui est inférieure à 2 µm et où la distance entre îlots est de 2 µm à 6 µm, les îlots d'activation étant entourés de zones hydrophobes non-activées, non-adhésives.

10. Méthode de la revendication 9 où la distance entre îlots est de 4 µm à 6 µm.

11. Méthode de la revendication 9 ou 10, où une activation par plasma localisée pour produire une pluralité de zones d'adhérence cellulaire hydrophiles activées par plasma ayant la capacité de réduire la réaction fibreuse est effectuée utilisant un pochoir ou masque ayant un réseau d'ouvertures en correspondance desdits îlots d'activation, ouvertures à travers lesquelles l'activation par plasma est appliquée à la surface traitée.

12. Méthode de la revendication 11 où le pochoir ou masque est mis(e) en contact avec la surface du dispositif à traiter pour l'activation.

13. Méthode de l'une quelconque des revendications 9 à 12, où avant l'implantation du dispositif, les zones d'adhérence cellulaire hydrophiles à surface oxydée en particulier activée par plasma, mais non pas les zones non-activées, sont couvertes de cellules autologues d'un sujet auquel la surface traitée doit être implantée.

14. Méthode de la revendication 13 où les cellules autologues appliquées incluent fibroblastes, cellules d'origine adipeuse, ostéocytes, chondrocytes, myocytes, cellules souches ou cellules dérivées du sang.
